(19) Europäisches Patentamt
European Patent Office
Office européen des brevets

(11) Publication number: **0 477 979 A2**

# EUROPEAN PATENT APPLICATION

(21) Application number: **91116576.9**

(22) Date of filing: **27.09.91**

(51) Int. Cl.⁵: **A61L 15/28**, A61L 27/00

(30) Priority: **28.09.90 JP 262204/90**

(43) Date of publication of application:
**01.04.92 Bulletin 92/14**

(84) Designated Contracting States:
**CH DE ES FR GB IT LI NL**

(71) Applicant: **SUNFIBRE CO.,LTD**
**133, Koyamacho-higashi 5-chome**
**Tottori-shi, Tottori(JP)**
Applicant: **PRESIDENT OF TOTTORI UNIVERSITY**
**Hayashi Shinzi, 101, Koyamacho-minami 4-chome**
**Tottori-shi, Tottori(JP)**

(72) Inventor: **Yoshihiro, Shigemasa**
**48, Mihagino 1-chome**
**Tottori-shi, Tottori(JP)**
Inventor: **Seiichi, Tokura**
**1-13, Hachikengojo-nishi 4-chome, Nishi-ku**
**Sapporo-shi, Hokkaido(JP)**
Inventor: **Akira, Matsuhashi, Tottori Daigaku Shirahama**
**Shukusha RA-12, 357 Koyamacho-nishi 1-chome**
**Tottori-shi, Tottori(JP)**
Inventor: **Saburou, Minami, Goudou Shukusha Koyamajuutaku**
**RCK-3-401, 251, Koyamacho-kita 3-chome**
**Tottori-shi, Tottori(JP)**
Inventor: **Hisato, Oota, c/o Sunfibre Co., Ltd.**
**133, Koyamacho-higashi 5-chome**
**Tottori-shi, Tottori(JP)**

(74) Representative: **TER MEER - MÜLLER - STEINMEISTER & PARTNER**
**Mauerkircherstrasse 45**
**W-8000 München 80(DE)**

(54) Biological filling agent and wound-healing agent.

(57) A biological filling agent and a wound-healing agent are provided. The biological filling agent includes a composite which is made of chitin or a derivative thereof, and a reinforcing material. The biological filling agent may further include an antibiotic. The wound-healing agent includes a cotton-like chitin product which is an aggregate of thread-like chitin; or a cotton-like chitosan product which is an aggregate of thread-like chitosan; or a sponge-like product which is made of chitin, chitosan and a derivative thereof. The biological filling agent and wound-healing agent are quite useful for the protection of a wound face and the filling of a tissue defect part or a dead cavity.

FIG 1

EP 0 477 979 A2

## BACKGROUND OF THE INVENTION

1. Field of the invention:

This invention relates to a biological filling agent for use in an organism and a wound-healing agent, which are prepared from chitin, chitosan and the like as a main component and which can be utilized for protecting the wound face of an organism and for filling the tissue defect part or dead cavity of an organism.

2. Description of the prior art:

Various types of therapeutic agents have been developed for the purpose of attaining an enhancement in wound healing, although the material used therefor is not limited to chitin and chitosan. Also a great number of materials have been proposed and marketed as a wound-covering protective agent for skin defect wounds such as thermal burns, skin-taken parts and skin-grafting parts for epidermis.

The wound-covering protective agents are classified mainly into those made of synthetic products and those made of natural products. Examples of the protective agents made of synthetic products are velvety sheets and woven fabrics made of nylon or polyester fibers, and porous sheets made of polyurethane or cross-linked polyvinylalcohol. Examples of the protective agents made of natural products are non-woven fabric cloths made of collagen (e.g., trade name Meipac, manufactured by Meiji Seiyaku Co.), frozen pig skins (trade name METASKIN, manufactured by Mitsui Seiyaku Co.), and fibrin films.

In recent years, much attention has been paid to various biological dressings derived from natural products because of their high compatibility with organisms and these biological dressings are used as a protective agent for skin defect wounds. However, such preparations do not completely fulfill their function as a wound-covering protective agent. For this reason, further investigations have been conducted on its material, shape and so forth.

Further, spraying agents such as bismuth subgallate, iodoform, and starch containing zinc oxide are used at present as a wound-covering protective agent. These agents are used for the purpose of not only protecting a wound face by covering but also promoting the wound healing by drying a moistening wound face with the use of their astriction or moisture absorption.

However, these agents do not have direct healing action or analgesic action themselves, and there is a danger that the use of these agents in large quantities will bring a disorder in the nervous system or digestive system as an adverse effect.

There are also antibacterial splaying agents such as Francetin T. However, these agents are used mainly for sterilization to infectious pathogens or for prevention of infection, and they do not always have a sufficient effect on the protection by covering or on the drying of a wound face. Also the application period and the amount of these agents are limited because of their tendency to cause anaphylaxis and their local stimulativity.

For antiphlogistic analgesic agents, there is cataplasm used as an antiphlogistic analgesic fixative agent. However, this agent cannot be applied to open wounds because of its strong stimulativity.

For vegetable antiphlogistic agents, there is a Melilotus officinalis extract used as a therapeutic agent for soft part tumescence. However, it has been observed that this agent has an adverse effect on the circulatory system or gastrointestine.

Further, conventional wound-healing agents are used for the purpose of removing any factors which inhibit the wound healing mechanism of an organism, and they are expected to have an indirect effect on the wound-healing mechanism. On the other hand, burying of a biological filling agent is required for the promotion of wound healing not only in the damage at the body surface but also in the abscess or the soft tissue defect caused by tumor extraction at the deep part in an organism.

However, conventional biological filling agents are used only for the orthopaedic therapy which utilizes artificial mammae, artificial noses or artificial eyes to maintain the shape against the particular soft tissue defect. As the substitute materials for maintaining the shape, synthetic products such as silicone, vinyl chloride and styrene foam are usually used. These materials are only buried in an organism to physically regulate the shape and they do not have any promoting action for wound healing. For the defect wound of soft tissue in the deep part in an organism, there are currently developed very few filling agents to be buried in a wound cavity for the promotion of wound healing. Only one example is a gelatin sponge (trade name Spongel, manufactured by Yamanouchi Seiyaku Co.; trade name Gelfoam, manufactured by Upjohn Co.) which is used as substitute for a hemostatic agent, although this is not suitable for use in large defect wounds which require filling or burying the agent. In particular, the inner part of a thigh, the axillary part and

2

the submandibular part have coarse subcutaneous tissue and also have the important nerves and the main artery, so that it is extremely difficult to close the wound cavity by suture. In a conventional treatment, drainage gauze is inserted into the wound cavity and there is no other way except intently waiting for the growth of granulation tissue. Accordingly, the therapeutic period is delayed, which imposes an extremely heavy burden on patients (including animals).

The conventional biological dressings such as a non-woven fabric cloth made of collagen and a frozen pig skin, which have been on the market as a wound-covering protective agent for skin defect wounds, do not have a sufficient function as a wound-covering protective agent.

One of the drawbacks thereof is that they do not have satisfactory adhesion to the wound face. In general, covering protective agents are required to have a function as an artificial skin to a certain extent, even if it is impossible to obtain those having a perfect function. That is, it is preferred that the protective agent functions in a body to the wound face during the treatment and tends toward the wound healing. If the protective agent has poor adhesion to the wound face, a great amount of exudates are retained at the interface of a disease part, so that the wound is very late in drying, resulting in a delay of epidermization.

Second, there is a drawback that the conventional protective agent can readily be decomposed at an early stage by the exudate. It goes without saying not only that the protective agent loses its function but also that the protective agent may become the cause of external infection to inhibit satisfactory epidermization.

Third, the conventional protective agent has poor capability of allowing the exudate produced at the wound face to move outside, so that the exudate has a tendency to stand at the diseased part, which may cause delayed healing of the wound face.

Fourth, there is no appropriate method for fixing a wound-covering protective agent to the wound part, and the protective agent can readily be removed therefrom. Therefore, it cannot be said that the protective agent exhibits its efficient action and effect.

For these reasons, the development of protective agents which are prepared from biological materials and reform the aforementioned drawbacks has been desired.

On the other hand, in the same manner as the case of a wound-covering protective agent for cutaneous damages, the defect wounds of soft tissue such as subcutaneous tissue and muscle in an organism, which can form a large dead cavity within the wound, should be healed by burying the wound cavity with a filling agent. In this case, because the filling agent is completely buried in the organism, it must have biocompatibility and must be organized as a substitute tissue. The filling agent is required to have the same characteristics as those of the damage-covering protective agent. Although the development of wound-covering protective agents is currently in progress, the development of filling agents to be buried in a wound cavity is also desired.

Because both chitin and chitosan fibers are biological materials and these fibers have excellent biocompatibility, it is expected that non-woven fabric cloths, woven fabrics and knit fabrics made of these fibers are favorable for use as a wound-face protective agent or a wound-healing promoting agent. However, the sheets made of chitin or chitosan have not always been considered to be an excellent wound-covering protective agent or wound-healing promoting agent in comparison with a conventional biotechnological dressing. For example, when a non-woven fabric cloth or sheet made of chitin or chitosan fibers is brought into contact with the wound part, there is a drawback that the contact face of the wound part is irritated to produce a great amount of exudates. Therefore, some holes may be made on the surface of the protective agent during the treatment, and the effects as a protective agent would be degraded, which is not preferred. Also when buried in the wound cavity for filling it, some disorder may be generated by retention of exudates.

As described above, in the clinical application of chitin and chitosan, the shape as a preparation and the like admit of improvement.

On the other hand, the application of a spraying agent to skin defect wounds and so forth has a wide adaptability, because the treatment using a spraying agent is simple. However, many problems have been pointed out in various aspects such as fixation and adhesion to the wound face, prevention of infection, and adverse effects caused by the use in large quantities.

The wound-healing agent for use in wounds is desired to the following advantages: (1) it is not irritative to the wound face; (2) it exhibits a protective effect by adhering to the wound face; (3) it allows the wound face to dry by absorbing an exudate; (4) it prevents the infection of the wound; (5) it has a direct wound-healing effect and a direct analgesic effect; (6) it has no adverse effects on an organism, and so forth.

**SUMMARY OF THE INVENTION**

The inventors have found that a wound-healing agent, which overcomes the drawbacks of the prior art and has the aforementioned advantages (1) to (6), can be obtained by using chitin, chitosan and derivatives thereof and making the most of their characteristics.

Thus, the invention described herein makes possible the objectives of providing a biological filling agent comprising a composite which is made of chitin or a derivative thereof and a reinforcing material; a biological filling agent comprising a composite which is made of chitin or a derivative thereof, an antibiotic and a reinforcing material; a wound-healing agent comprising a cotton-like chitosan product which is an aggregate of thread-like chitosan; a wound-healing agent comprising a sponge-like product which is made of chitin, chitosan and a derivative thereof; and a wound-healing agent comprising a cotton-like chitin product which is an aggregate of thread-like chitin.

## BRIEF DESCRIPTION OF THE DRAWINGS

This invention may be better understood and its numerous objectives and advantages will become apparent to those skilled in the art by reference to the accompanying drawings as follows:

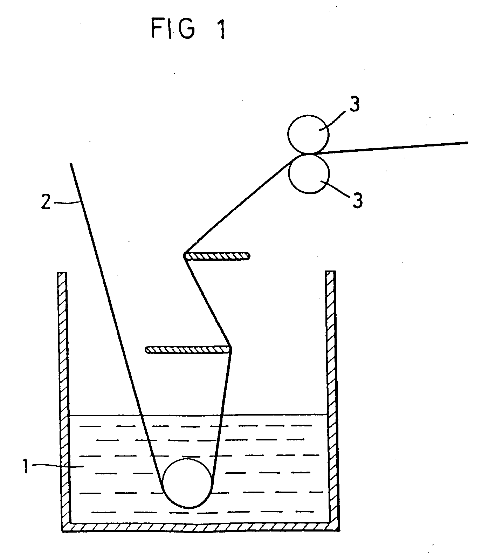

Figure 1 is a schematic diagram showing an apparatus used in the preparation of a biological filling agent according to this invention.

## DETAILED DESCRIPTION OF THE INVENTION

The biological filling agent and the wound-healing agent of this invention are made of chitin and chitosan which are biological materials.

Chitin which is used in this invention is a polysaccharide having as a constituent unit, N-acetyl- D-glycosamine which is widely distributed in nature and contained in the Crustacea such as shrimps and crabs; insects such as grasshoppers and beetles; cuttlebones and the like. Chitin has a structure similar to that of cellulose. As can be estimated from such a structure, chitin is chemically stable and does not react with almost all of the reagents under mild conditions. Also appropriate solvents which can dissolve chitin under mild conditions have not been found so far. For this reason, chitin is hard to deal with and therefore little use is found in the existing circumstances. In recent years, however, it has been suggested that an antitumor agent containing chitin as an active ingredient has an excellent antitumor activity (e.g., Japanese Patent Publication No. 59-27826).

Moreover, chitin and derivatives thereof have high lysozyme receptivity, excellent digestibility in an organism, biocompatibility, familiarity at the cell level, high permeability of intermediate molecular weight substances in sera, and great capability of adsorbing blood components such as serum proteins. In addition, it has been expected that immunogenicity becomes higher with an increase in the degree of deacetylation of chitin. It has also been known that chitin subjected to deacetylation at a ratio of 70% exhibits the greatest capability of activating macrophages.

The term chitin used in this invention includes chitin subjected to deacetylation at a ration of 50% or lower, in addition to chitin obtained by purification of natural products. Various chitin derivatives obtained by chemical conversion of chitin are also included therein. As the chitin derivatives, there can be mentioned, for example, alkylchitins such as carboxymethylchitin and hydroxyethylchitin, acetylchitin, and sulfurylchitin. Examples of the esterification products of chitin are those obtained from carboxylic acids such as formic acid, acetic acid, propionic acid, butyric acid, valeric acid, isopropionic acid, isobutyric acid, isovaleric acid, benzoic acid, cinnamic acid, salicylic acid, anthranilic acid and phthalic acid; sulfonic acids such as sulfuric acid, toluenesulfonic acid and sulfanilic acid; carbonic acids; and anhydrides thereof.

In this invention, chitosan which is a deacetylation product of chitin also falls under the category of chitin derivatives.

As for chitosan which is obtained from chitin by deacetylation, it has been suggested in Japanese Laid-open Patent Publication No. 56-26049, No. 59-88424 and No. 62-170254 that chitosan is effective as a promoting agent for wound healing. Moreover, chitosan thin films have a property of transmitting water and low molecular weight substances; they also have good compatibility with any kind of biological tissue and do not cause tissue reaction. The chitosan thin films have a function as a biomaterial and many attempts have been made at applying them to microcapsule materials, dialysis membranes, artificial organs, operation materials, blood vessel reinforcing materials and so forth.

As seen from the foregoing, as a substitute material for defect wounds of tissue at the deep part, it is expected that a composite of chitin or chitosan and a reinforcing material having suitable elasticity, strength and hardness is a more effective biological filling agent.

The reinforcing material which can be used in this invention is not particularly limited, so long as it not only has a physical function such as substance separation and structure support, non-toxicity and sterilizability, but also does not cause rejection of an organism; when a biological filling agent, comprising a composite which is made of the reinforcing material and chitin or a derivative thereof, is used for medical applications, the reinforcing material exhibits preferable strength, flexibility, hardness and shape for the operation site. In particular, preferably used are non-woven fabric cloths such as those made of polyester, polypropylene, cellulose or the like.

The treatment using a wound-healing agent which contains chitin or chitosan as a main ingredient according to this invention has many advantages that the treatment conditions (e.g., frequency and amount) can be adjusted at any time depending upon the shape and degree of wounds; the retention of an exudate as an inhibiting factor for wound healing can therefore be prevented, which is effective for the drying of the wound face; it makes possible the accurate prognostic judgment because the wound face can be observed by looking straight thereat, and so forth.

Moreover, chitin and chitosan have themselves extremely excellent action for the growth of granulation tissue and therefore exhibit a direct promoting effect on the healing mechanism. This action is not found in any conventional wound-healing agents and is the most important feature of chitin and chitosan as a wound-healing agent. Another important feature is that chitin and chitosan have a capability of removing a plurality of inhibiting factors for the wound-healing mechanism by their bactericidal action, local antiphlogistic and analgesic action, lytic and absorptive action to necrotizing tissue, and so forth. Further, chitin and chitosan can find extremely wide applications as compared with conventional wound-healing agents, and it can be said that they are effective medicaments, in view of the advantages that they have compatibility with an organism and are non-toxic; they are not irritative; and it is possible to directly use chitin or chitosan itself which was prepared into a cotton-like, flake-like or sponge-like shape.

**DESCRIPTION OF THE PREFERRED EMBODIMENTS**

The invention will be further described by reference to the following embodiments which are intended to illustrate, but not limit, the invention.

Embodiment 1

In this embodiment, various methods for the preparation of a biological filling agent is first explained.

(1) First, 0.1 to 10 g (dry weight) of purified chitin was disaggregated (dispersed and swollen) in 50 ml of water at 40°C or lower by use of a homogenizer or a mixer. The mixture was poured into a predetermined amount of water or an organic solvent, for example, an alcohol such as methanol and ethanol, or an acetone, to achieve 0.5 to 4.0 g of chitin per liter. The chitin suspension obtained was poured in about 200 ml portions onto a reinforcing material (a non-woven fabric cloth made of polyester) with a thickness of 0.1 to 5.0 mm by use of a suction-type paper-making apparatus of the batch style, resulting in a composite sheet having a chitin layer. (The thickness of the composite sheet may be adjusted by an original concentration of chitin.)

The composite sheet thus obtained was sucked by use of a vacuum pump from the opposite side to the surface of the chitin layer made on the reinforcing material. According to this suction, the adhesiveness and the degree of intertwining between the chitin layer and the reinforcing material were increased. Thereafter, the composite sheet was dried at 10°C to 80°C by putting it between the leaves of hygroscopic material such as filter paper, resulting in a filling agent for use in an organism.

(2) First, purified chitin was dissociated in water at 40°C or lower by use of a homogenizer or a mixer to prepare a 0.5% to 8.0% chitin suspension.

A reinforcing material with a thickness of 1.0 to 5.0 mm was impregnated with the chitin suspension thus prepared, and freeze-dried (when necessary, these steps may be repeated two or three times), resulting in a sponge-like biological filling agent.

(3) As shown in Figure 1, using an apparatus where an aqueous suspension **1** of purified chitin powder at a concentration of 0.1% to 12.0% was contained in a bath, a reinforcing material **2** (a non-woven fabric cloth made of polyester) with a thickness of 1.0 to 5.0 mm was continuously allowed to pass through the suspension **1** to apply the suspension **1** to both surfaces of the reinforcing material **2**. The reinforcing material **2** thus treated was rolled by a pair of rollers **3**, and then dried at 100°C to 130°C, resulting in a biological filling agent.

(4) First, purified chitin was disaggregated in water at 40°C or lower by use of a homogenizer or a mixer to prepare a chitin suspension with a chitin concentration of 0.5% to 3.0%.

The chitin suspension thus prepared was added to an aqueous solution of chitosan lactate or acetate with a concentration of 0.01% to 0.5% to achieve a chitin concentration of 0.01% to 0.4%, resulting in a formulated concentrate for paper making.

Then, using a continuous paper-making apparatus, chitin paper was made on the surface of a reinforcing material (a non-woven fabric cloth made of cellulose) with a thickness of 0.05 to 1.0 mm, resulting in a biological filling agent.

(5) The same apparatus was used as in the method (3). First, chitosan obtained by deacethylation of chitin was suspended in water at a concentration of 0.1% to 12.0%, and the aqueous suspension was poured into a bath of this apparatus. Then, a reinforcing material (a non-woven fabric cloth made of cellulose) with a thickness of 1.0 to 5.0 mm was continuously allowed to pass through the suspension to apply the suspension to both surfaces of the reinforcing material. The reinforcing material thus treated was dried at 100°C to 130°C while being rolled, resulting in a biological filling agent.

(6) First, purified chitosan was dissolved in a 2% to 6% aqueous solution of acetic acid to obtain a 0.1% to 0.3% solution of chitosan. This solution was mechanically applied to a reinforcing material (a non-woven fabric cloth made of cellulose) flowing at a constant rate. Thereafter, this reinforcing material was allowed to pass through a KOH-ethyleneglycol mixture at a constant rate for fixation.

Next, the reinforcing material was allowed to pass slowly through a 40-60% ethanol-water mixture and then deionized water for repeated washing. Finally, the reinforcing material was dehydrated in ethanol, and dried with air, resulting in a biological filling agent.

(7) By use of a homogenizer or a mixer, purified chitin was disaggregated at 40°C or lower as a 0.5% to 8.0% chitin suspension to prepare a viscous chitin suspension in advance.

To 500 ml of this viscous chitin suspension, 5 to 100 g of oxytetracycline as an antibiotic was added. Then, a reinforcing material with a thickness of 1.0 to 5.0 mm was impregnated with this mixture, and freeze-dried (when necessary, these steps may be repeated two or three times), resulting in a sponge-like biological filling agent.

(8) Each of the composites (biological filling agents) obtained in the method (1) to (6) was further impregnated by spraying with a 1% to 10% aqueous solution or water-ethanol mixed solution of chitin and chitosan oligomers or antibiotics, resulting in a biological filling agent.

The biological filling agents obtained by the aforementioned various methods according to this invention are preferably used at an operation site after sterilization. As a method for sterilization, for example, conventional steam sterilization or ethylene oxide gas sterilization (EOG sterilization) can be used.

Also it is effective that the biological filling agent according to this invention is impregnated with or allowed to contain oxytetracycline (OTC) and other antibiotics, as described above.

In this invention, chitin powder is pulverized into uniform particles, and dispersed in water, after which the dispersion is freeze-dried and subjected to stem sterilization, resulting in a sponge-like chitin product. If the sponge-like chitin product thus obtained is used alone without making a composite with a reinforcing material, the expected advantages can be attained in some cases.

Moreover, it is possible to promote the healing effect much more by further spraying chitin or chitosan oligomers onto the biological filling agent obtained in this invention.

Embodiment 2

The following will describe another embodiment of this invention.

First, 1.5 g of chitin powder (manufactured by Nihon Suisan Co.) obtained from cuttlebones was suspended in 1 liter of water to form a chitin suspension. The chitin suspension obtained was poured onto a non-woven fabric cloth made of polyester as a reinforcing material, resulting in a composite sheet having a chitin layer. The composite sheet thus obtained was sucked by use of a vacuum pump from the opposite side to the surface of the chitin layer made on the reinforcing material. According to this suction, the adhesiveness and the degree of intertwining between the chitin layer and the reinforcing material were increased. Thereafter, the composite sheet was dried at 20°C to 30°C by putting it between the leaves of filter paper, resulting in a biological filling agent **A**.

The clinical examples using the biological filling agent **A** and the therapeutic effects thereof will be described below.

(1) The biological filling agent obtained above was used as a filling agent for the opening formed by perineal hernia of a canine. The perineal region was incised and the hernial opening was confirmed. The filling agent was wound into a roll form so as to fit with the wound cavity, and inserted into the wound cavity along the running of the rectum. The filling agent was sutured with two or three stitches on the perineal muscle membrane, coccygeal muscle and anal sphincter muscle, on which the subcutaneous tissue was subjected to buried suture, and finally the skin suture was conducted to complete the operation. The operation time was shortened to 30 minutes or less.

Thus, the hernial opening disappeared by filling it with the biological filling agent according to this invention. Also the running linearity of the rectum in the pelvic cavity was recovered.

Immediately after the operation, the tenesmus at the time of evacuation and the constipation, which had been observed before the operation, disappeared and healed. Moreover, no systemic reaction or skin reaction caused by the insertion of the filling agent was observed.

(2) The biological filling agent was buried in the subcutaneous portion as it had a sheet form, as a reinforcing material for the stitches of bovine umbilical hernia, and the peripheral portion of the sheet was fixed by interrupted suture on the straight muscle of abdomen.

In the operation of bovine umbilical hernia, the split of stitches by abdominal pressure was often observed. In contrast, the progress of healing in the aforementioned application was satisfactory, and the healing up of the skin stitches at the first stage was observed. Thus, it is considered that the biological filling agent according to this invention can be expected to have a subsidiary effect on the prevention of a split in the suture wound.

(3) The biological filling agent was used as a tendon substitute in the elongation technique for musculus flexor digitorum profundus against the spherical apophysis occurring at the inner part of a bovine thigh. When the musculus flexor digitorum profundus was cut off and the fetlock was elongated to the normal position, the cutting parts were separated proportionally to the degree of contraction of the musculus flexor digitorum profundus. The biological filling agent having a rectangular form was folded double, both ends of which were fixed to the musculus flexor digitorum profundus by the closed-loop suture using #2 nylon threads. The fascia, subcutaneous tissue and skin were closed by suturing in the usual manner, and the stitches were externally fixed with a casting tape for a week, thereby attaining a complete healing.

Thus, it was proved that the biological filling agent according to this invention has the advantage of being useful as a tendon substitute.

(4) The dehorning of cattle was conducted for the purpose of beauty. The biological filling agent was inserted into the subcutaneous portion as it was in a sheet form, and the skin was sutured. After two weeks, however, the skin was split and the filling agent was exposed. It is considered that the skin was split because the skin at the dehorned site had no margin and the tension of the skin was further increased by insertion of the filling agent. Moreover, no inflammatory response to the filling agent was observed on the inside of the wound.

(5) In order to confirm the effect of the aforementioned biological filling agent **A**, the biological filling agent **A** and a non-woven fabric cloth made of polyester were respectively buried in the four subcutaneous portions of the dorsolumbar region and lumbosacral region of each of six mongrel adult dogs (having a weight of 8 to 10 kg). On the second, fourth, eighth and eighteenth days after the operation, the buried portions were cut away and subjected to histological observation. Also the secrete obtained at that time was examined by microscopy using the Giemsa staining method.

In the period of examination, macrophages were activated and fresh neutrophilic leukocytes (one kind of the leukocytes) were observed in every cases. More significant effect was exhibited in the case where the biological filling agent **A** according to this invention had been used.

The adhesion between the reinforcing material and the subcutaneous tissue made steady progress day by day. When the filling agent had been used, the organization and the vascularization of intermediate arteries and veins in the reinforcing material were satisfactorily observed. In contrast, when only the non-woven fabric cloth made of polyester had been used, the granulated portion assumed an anemic color, and the formation of blood capillaries and the growth of fibroblasts were both poor. Moreover, when the filling agent had been used, the growth and activation of fibroblasts were particularly significant in the peripheral granulation tissue. These clinical examples and the healing effects thereof are listed in Table 1 below.

## Table 1

(1) Kind of biological filling agent used; Animals for clinical treatment; Diseases; Therapeutic effects

(2) 1; Biological filling agent A; Canine; Perineal hernia; +

(3) 2; ditto; Cattle; Umbilical hernia; +

(4) 3; ditto; ditto; Spherical apophysis; +

(5) 4; ditto; ditto; Dehorning; -

(6) 1; Biological filling agent B; Feline; Caudal bite;-

(7) 2; ditto; ditto; Facial bite; +

(8) 3; ditto; ditto; Subcutaneous abscess; ±

(9) 4; ditto; ditto; ditto; ±

(10) 5; ditto; Canine; Mammary tumor; +

(11) 6; ditto; ditto; Testicular tumor; +

(12) 7; ditto; ditto; Dorsal bite; +

(13) 8; ditto; ditto; Monkey; Facial bite; +

(14) 1; Biological filling agent C; Feline; Subcutaneous abscess; ±

(15) 2; ditto; ditto; ditto; ±

(16) 3; ditto; Rabbit; Mandibular abscess; +

+: Extremely favorable healing was obtained.

±: After operation, the retention and outflow of a blood-like exudate were observed, however, inflammatory images were not observed with the naked eye and satisfactory granulation tissue was grown.

-: No effects were observed.

The therapeutic effects shown in Table 1 were determined on the following bases.

First, 20 g of chitin powder (manufactured by Nihon Suisan Co.) obtained from cuttlebones was pulverized into uniform particles with a size of 20 to 40 mesh and suspended in 0.5 liter of water to form a chitin suspension. A non-woven fabric cloth made of polyester and rayon (with a thickness of 2 mm and a diameter of 6 cm) was impregnated with 250 ml of the chitin suspension and freeze-dried. These steps were repeated twice, and the product obtained was subjected to steam sterilization at 120°C, resulting in a biological filling agent **B**.

The clinical examples using the biological filling agent **B** and the healing effects thereof are shown in Table 1 above. In Table 1, among examples No. 1-8, examples No. 5 and 6 indicate the cases of a closed clean wound, whereas the others indicate the cases of an open stained wound. In the cases of a clean wound, the biological filling agent **B** in several layers was buried in the dead cavity so that the dead cavity was completely filled therewith, and the subcutaneous tissue was sutured thereon. Therefore, the buried suture at the filled part was formed by several layers.

Both of examples No. 5 and 6 were the cases of a canine malignant tumor (No. 5: malignant mixed tumor, No. 6: Sertolis's cellular tumor), and the healing up of the skin at the first stage was observed in either case. In examples No. 1-4, 7 and 8, the wound was opened with a knife; in the cases of an abscess, the drain of pus was conducted and the wound cavity was washed with Oxyful and Hibidil. Then, sponge-like chitin was torn into pieces with an appropriate size and filled in the wound cavity by use of a hemostatic forceps and a grooved director.

In examples No. 2, 7 and 8, by only one insertion, granulation tissue was grown in the wound for 10 to 14 days, and the formation of a skin was observed, which brought wound healing.

In examples No. 3 and 4 which are the cases of a feline subcutaneous abscess, immediately after the insertion of sponge-like chitin, exudation was stopped, however, another exudation was observed in 5 to 7 days. However, this exudation was considered to be not inflammatory but a response to the biodecomposition of chitin, and favorable growth of granulation tissue was observed in the wound cavity. Several times insertion of sponge-like chitin was required for wound healing. On the other hand, no contraction of the skin covering the wound cavity was observed through the healing period. In the case of a feline caudal bite, the wound reached the coccygeal bone, and the formation of an exudate could not be stopped only by the filling of sponge-like chitin. This is because the feline tail had high mobility and external force was always applied to the filled sponge-like chitin. It seems that the healing effect of the wound at the mobile site is reduced.

The biological filling agent **C** was obtained in the same manner as described above, except that 40 g of oxytetracycline (OTC) as an antibiotic was dispersed in a dispersion of chitin powder used in the production of the biological filling agent **B**.

This biological filling agent **C** containing an antibiotic was used in the clinical examples No. 1 to 3 shown in Table 1.

Examples No. 1 and 2 are the cases of a feline subcutaneous heavy abscess where after the drain of pus and then washing, the biological filling agent **C** was cut into strips and filled in the wound. When the biological filling agent **C** was used, the period until the occurrence of another exudation was prolonged as compared with the biological filling agent **B**. Although the effect lasted for 10 to 14 days, another exudation was observed similarly to the case where the biological filling agent **B** was used, and the healing by an insertion could not be expected. There was no difference in the ability of promoting the growth of granulation tissue between the biological filling agents **B** and **C**.

Moreover, example No. 3 is the case of a rabbit mandibular abscess where after the drain of pus and then washing, the biological filling agent **C** was cut into strips and filled in the wound. After about two weeks, another exudation was not observed, whereas favorable growth of granulation tissue was observed.

Embodiment 3

The following will describe still another embodiment of this invention.

(1) Preparation of a cotton-like chitosan product

Chitosan threads spun out of chitosan were washed with running water for a night or longer, and then with hot water (60°C to 90°C) for 2 to 7 hours, after which these threads were immersed in a volatile solvent which is satisfactorily miscible with water, such as methanol, ethanol and acetone, for a whole day and night or longer. The threads cut in a length of about 1 to 4 cm were treated in a mixer and then dried, resulting in a cotton-like chitosan product.

The cotton-like chitosan product obtained was composed of fibers 2 to 20 mm in length, 20 to 50 $\mu$m in

width and 3 to 15 μm in thickness, and had an apparent specific gravity of 0.01 to 0.2 g/cc.

Example 1: 200 g of chitosan (Flonac-C, having a diameter of 50 to 60 μm) manufactured by Kyowa Yushi Kogyo Co. was dissolved in a mixture of water and acetic acid in a volume ratio of 23:1) by stirring and filtered twice by the application of pressure (1 to 2 kg/cm$^2$), followed by standing overnight for defoaming. The formulated concentrate was spun by extruding from a nozzle having 500 holes 0.1 mm in diameter into a mixed solution containing 10 liters of ethyleneglycol, 1.5 kg of ice and 1.8 kg of potassium hydroxide, after which the coagulation of the spun threads was allowed to further proceed in a mixed solution of methanol and water (1:1). Then, these threads were 1.15-fold stretched in air and washed with water overnight, after which these threads were treated with hot water at 70°C to 80°C for 3 to 5 hours and immersed in methanol overnight, followed by reeling with a reeling machine and natural drying. The chitosan threads obtained were cut in a length of 1 to 2 cm and treated with mixer, resulting in a cotton-like chitosan product.

The cotton-like chitosan product had an apparent specific gravity of 0.026 g/cc and was composed of fibers 9 mm in length, 26 μm in width and 9 μm in thickness.

Example 2: The chitosan threads spun under the same conditions as used in Example 1 were reeled on a bobbin and washed with running water overnight. After washing with hot water at 70°C to 80°C for 3 to 5 hours, these threads were immersed in methanol for a whole day and night or longer. The threads were removed from the bobbin and cut in a length of about 2 cm. The cut threads were treated with a mixer.

The clinical examples using the cotton-like chitosan product prepared in Example 1 above are shown in Table 2 below. The therapeutic effects in Table 2 were determined in the same manner as that described in the footnote of Table 1.

## Table 2

(1) Examples; Animals; Diseases; Therapeutic effects

(2) 1; Canine (female, mongrel); Gangrenous mastitis; (+)

(3) 2; Feline (female, mongrel); Bite; (+)

(4) 3; Feline (female, mongrel); Bite (tail); (+)

(5) 4; Feline (female, mongrel); Bite (hind-leg); (+)

(6) 5; Feline (male, mongrel); Contusion-Bone fracture (fore-leg); (+)

(7) 6; Cattle (female, domestic); Coronary dermatitis; (+)

(8) 7; Equine (male, half-blooded ); Hoof cancer; (+)

(9) 8; Cattle (female, domestic); Laceration; (+)

(10) 9; Feline (male, Persian); Contusion-Dislocation (hind-leg); (-)

(11) 10; Feline (male, Persian); Amputation; (+)

(12) 11; Cattle (male, F1); Abrasion; (+)

(13) 12; Feline (female, mongrel); Infection of operative wound;(±)

(14) 13; Feline (female, mongrel); Infection of operative wound; (+)

(15) 14; Feline (female, mongrel); Infection of operative wound; (+)

(16) 15; Cattle (female, Holstein); Abscess; (+)

(17) 16; Cattle (female, Holstein); Abscess; (+)

(18) 17; Cattle (female, Holstein); Hoof bottom ulcer; (+)

(19) 18; Cattle (female, Holstein); Hoof bottom ulcer; (+)

(20) 19; Cattle (female, Holstein); Hoof bottom ulcer; (+)

11

As shown in Table 2, the cotton-like chitosan product was used mainly as a wound-healing agent for filling in the external wounds and operative wound. The items were two examples of canines, eight examples of felines, eight examples of cattle and one example of an equine with the total thereof being nineteen examples. The application effects were as follows; satisfactory reaction was observed for seventeen examples in nineteen examples. In one example (No. 9) which was the case of a feline contusion, no tissue reaction accompanying the use of a cotton-like chitosan product was observed. However, in this example, peripheral arteries were torn and blood circulation in the diseased part was interrupted by a heavy tissue damage due to a traffic accident and by a physical rotation of the diseased part due to a dislocation of the hock part. An example showing the symptoms which formed a striking contrast to this example was the case of feline No. 5. In the example, a heavy tissue damage due to a traffic accident and a fracture of the paw's middle-digit born were observed; however, the blood circulation toward the end of digits was maintained at the contralateral side of the damage site, and extremely satisfactory reaction was observed to the cotton-like chitosan product. In example No. 9, the contused site was amputated and the fresh wound was filled with a cotton-like chitosan product, whereby satisfactory healing of the wound was observed. From these facts, blood circulation is needed for the application of the present agent and the regeneration of extinct tissue is not expected.

In three examples (Nos. 12 to 14), the filling of the present agent was applied to a split in the suture wound, which was caused by the infection of an operative wound. The results had a tendency that a significant effect was obtained in the case of a feline. A similar effect was also obtained for cattle and equine. In two examples, a surface abscess of dairy cattle which has been scarcely considered to be an object of treatment so far was completely healed by only one filling of the present agent. Further, an ulcer of the hoof bottom of dairy cattle was completely healed by only one filling and pressure-fixing of the present agent. One example for equine was the case of a disease, malignant chronic dermatitis, which can never be expected to heal such that it is called hoof cancer as another name. However, by surgical excision of the diseased part and by filling and pressure-fixing of the present agent in the defect part, extremely satisfactory walking on the ground was possible in the next day, after which no relapse was observed and complete healing was attained. Moreover, after healing, no sclerotization or functional disorder caused by cicatrization of the diseased part was observed in any case.

As mentioned above, the wound-healing agent of this invention is considered to be a therapeutic agent having wonderful capability, because one to several applications of this agent to a diseased part brought about satisfactory tissue-recovering capability in a patient such as a canine, feline, cattle and equine, and it was possible to completely heal a chronic disease which had been considered to be incurable so far.

(2) Production of a sponge-like product made of chitin, chitosan and derivatives thereof

Chitin, chitosan and derivatives thereof, or a mixture of these materials and a water-soluble polymer, such as polyvinyl alcohol, polyacryl amide, starch and gelatin, which was added thereto, were treated in a mixer for 0.5 to 2 hours to form a 0.1% to 5% (w/v) dispersion. Only the dispersion, or a composite of felt- or sponge-like polyester or polyurethane and the dispersion prepared, was frozen at -10°C to -40°C. The frozen material was freeze-dried, resulting in a sponge containing chitin, chitosan and derivatives thereof.

Example 1: Using a mixer, a 1.5% (w/v) dispersion of cuttlebone chitin in water was prepared. Then, 5.5 ml of the dispersion prepared was put into a petri dish (40 mm in diameter and 5 mm in height) made of polystyrene, and frozen at -20°C, followed by freeze-drying for a whole day and night, resulting in a sponge-like chitin product.

Example 2: First, a 2% (w/v) dispersion of cuttlebone chitin in water was prepared. Then, felt is cut into a circle having a diameter of 5 cm and placed on the bottom of a glass vessel. To this, 15 ml of the dispersion was added, and the circular felt was impregnated with the dispersion, followed by freeze-drying at -20°C. Another felt was placed on the frozen felt, which were impregnated with 15 ml of the dispersion and then frozen again at -20°C. The frozen felt composite was freeze-dried for a whole day and night, resulting in a sponge-like chitin product.

The clinical examples using the sponge-like chitin product prepared in Example 1 above are shown in Table 3 below. The therapeutic effects in Table 3 were determined in the same manner as that described in the footnote of Table 1.

## Table 3

(1) Examples; Animals; Diseases; Therapeutic effects

(2) 1; Canine (female, Maltese); Gingivitis; (+)

(3) 2; Feline (female, mongrel); Bite; (+)

(4) 3; Feline (female, Siamese); Bite; (+)

(5) 4; Feline (female, mongrel); Bite (tail); (+)

(6) 5; Cattle (male, domestic); Arthritis; (+)

(7) 6; Canine (female, mongrel); Infection of operative

    wound; (±)

(8) 7; Canine (male, Maltese); Gingivitis; (+)

As shown in Table 3, the sponge-like chitin product was used mainly in seven examples of infectious wounds. The items were three examples of canines, three examples of felines and one example of cattle. The effects of the application were observed in six examples out of seven. In one split example of a skin suture wound, which was caused by infection of an operative wound, the effect was uncertain. In this example, it was considered that favorable growth of granulation tissue was not caused because there remained several sutures which had been buried under the skin by another operation and these sutures were nonabsorbable.

When the present agent was used for local filling in two examples (Nos. 1 and 7) of canine periodontosis, favorable growth of granulation tissue was observed and these wounds were completely healed. Also when the present agent was used for filling in one example (No. 5) of cattle arthritis, favorable recovery of tissue was observed. For external wounds Nos. 2 to 4, extremely favorable healing mechanism was achieved by one to several fillings of the present agent. As mentioned above, the present agent is considered to be an ideal wound-healing agent because it can be applied not only to external wounds but also to intraoral diseases and no adverse effects are observed.

Furthermore, the sponge-like chitin product produced in Example 1 was used in two examples of canines. One example was for filling in the tissue defect part caused by castration, and the other was for filling in the crack of a fractured bone. In both examples, no adversely effects were observed and these wounds were completely healed. In particular, it was considered that the promotion of regenerating capability of bone tissue was expected in the case of a bone fracture.

## Table 4

(1) Examples; Animals; Diseases; Therapeutic effects

(2) 1; Canine (male, mongrel); Castration; (+)

(3) 2; Canine (male, Dorgo); Bone fracture; (+)

(3) Production of a cotton-like chitin product

The cotton-like chitin product was produced by pulverizing chitin threads, granules or flakes with the use of a pulverizer. The pulverization was conducted using hammers and liners of various shapes at a rotational frequency of 1000 to 9000 rpm with a rated current of 20 to 30 A. The obtained cotton-like chitin had an apparent specific gravity of 0.01 to 0.3 g/cc and was in the form of fibers 0.1 to 10 mm in length and 5 to 400 $\mu$m in width.

Example 1: As the pulverizer, ACM Pulverizer 10 manufactured by Hosokawa Micron Co. was used, 3.6 kg of cuttlebone chitin was pulverized using a bar-shaped hammer and a grooved liner for 25 minutes at a rotational frequency of 6800 rpm with a rated current of 24.2 A. The obtained cotton-like chitin had an apparent specific gravity of 0.05 to 0.129 g/cc and was composed of fibers 200 to 800 $\mu$m in length and 10 to 120 $\mu$m in width.

An experiment of local reaction caused by burying under the skin of the cotton-like chitin product obtained in

Example 1 was conducted using the following animal.

Animal: canine, Species: mongrel, and Age: two years.

After an abscess was experimentally formed with the use of staphylococci, the cotton-like chitin product was filled in the diseased part and the clinical progress was observed. A pain of the diseased part disappeared on the first day after the filling, and tumescence and flare disappeared up to the fifth day. Also a general symptom was remedied on the third day after the filling.

As described above, the biological filling agent of this invention can attain excellent advantages that when buried in a wound cavity no inflammatory images are observed by the naked eye according to the effect of chitin and a derivative thereof, and that favorable growth of granulation tissue is exhibited.

The biological filling agent of this invention can also attain excellent advantages that when buried in a wound cavity various cells as a biophylaxis function are allowed to migrate for the purpose of treating the staining matter and necrotizing tissue in the wound, and that fibroblasts are stimulated to increase the number thereof and extremely favorable granulation tissue is developed together with the development of a blood vessel system. As a matter of course, the activation of fibroblasts and the development of blood vessels enhance the skin healing. Moreover, in the case of a split wound, a conventional therapeutic method immediately causes the atrophy of skin, whereas the application of the present agent causes almost no atrophy of skin and has the advantage that the wound can readily be sutured after cleaning.

In addition, the biological filling agent has extremely excellent characteristics as a filling agent used for defect parts, because the organization of polyester is immediately caused by invasion of fibroblasts and blood vessels. When applied to clinical examples, significant effects were observed in thirteen examples out of fifteen. In the remaining two examples, favorable reaction was observed in granulation. The biological filling agent is completely harmless to an organism. In this respect, it can be said that the biological filling agent is an extremely excellent filling agent.

In the case of a skin erosion and ulcer, a great deal of pain is given, because the sensory part is exposed. For the treatment of these diseases, a wound-healing agent is required which gives a pain relief and immediate growth of granulation tissue. The wound-healing agent of this invention gives no pain when covering the wound and forms a coating which protects the wound to exhibit an analgesic effect. With the use of the wound-healing agent, vigorous growth of granulation tissue is observed and the normalization of the wound is allowed to significantly proceed by the ability to make an activation of macrophages and a migration of neutrophilic leukocytes, which is peculiar to chitin material. Moreover, unnecessary scars are scarcely formed, and from the viewpoint of cosmetic surgery, it is considered that the wound-healing agent has all the ability of a wound-covering agent.

The cotton-like or sponge-like wound-healing agent of this invention can be applied to various wound cavities regardless of their shapes, and the amount of agents to be filled can readily be controlled. When the wound-healing is buried in the wound cavity, no inflammatory images are observed by the naked eye according to the effect of chitin. Moreover, various cells as a biophylaxis function are allowed to migrate for the purpose of treating the staining matter and necrotizing tissue in the wound, and fibroblasts are stimulated to increase the number thereof, and extremely favorable granulation tissue is developed together with the development of a blood vessel system. As a matter of course, the activation of fibroblasts and the development of blood vessels enhance the skin healing. In the case of a split wound, a conventional therapeutic method immediately causes the atrophy of skin, whereas the application of the present agent causes almost no atrophy of skin and is excellent in the biological filling effect. Furthermore, granulation tissue formed by the present wound-healing agent contains a great number of blood capillaries and the atrophy of surrounding skin is difficult to occur. From this point, not only the cleaning of a stained wound by covering or filling but also the narrowing of the wound cavity by formation of benign granulation tissue is first achieved, and an extremely satisfactory healing effect by secondary closing is expected for cases where temporally closing is difficult. When the wound-healing agent is made of a cotton-like chitosan product or a cotton-like chitin product, it is particularly useful for cases where wounds are externally covered thereby. When the wound-healing agent is a sponge-like product made of chitin, chitosan and derivatives thereof, it is particularly useful for cases where a cavity formed by removal of a tumor is filled therewith.

It is understood that various other modifications will be apparent to and can readily be made by those skilled in the art without departing from the scope and spirit of this invention. Accordingly, it is not intended that the scope of the claims appended hereto be limited to the description as set forth herein, but rather that the claims be construed as encompassing all the features of patentable novelty that reside in the present invention, including all features that would be treated as equivalents thereof by those skilled in the art to which this invention pertains.

**Claims**

1. A biological filling agent comprising a composite which is made of chitin or a derivative thereof and a reinforcing material.

2. A biological filling agent comprising a composite which is made of chitin or a derivative thereof, an antibiotic and a reinforcing material.

3. A wound-healing agent comprising a cotton-like chitosan product which is an aggregate of thread-like chitosan.

4. A wound-healing agent comprising a sponge-like product which is made of chitin, chitosan and a derivative thereof.

5. A wound-healing agent comprising a cotton-like chitin product which is an aggregate of thread-like chitin.

6. A process for the manufacture of the biological filling agent according to claim 1, **characterized by** impregnating the reinforcing material with chitin or a derivative thereof.

7. A process for the manufacture of the biological filling agent according to claim 2, **characterized by** impregnating the reinforcing material with chitin or a derivative thereof and an antibiotic.

8. A process for the manufacture of the wound-healing agent according to claim 3, **characterized by** spinning chitosan to provide threads which are cut, treated in a mixer and then dried.

9. A process for the manufacture of the wound-healing agent according to claim 4, **characterized by** freeze drying an aqueous dispersion of chitin, chitosan and derivatives thereof, or a mixture of these materials and a water-soluble polymer, or a composite of felt- or sponge-like polyester or polyurethane and said dispersion.

10. A process for the manufacture of the wound-healing agent according to claim 5, **characterized by** pulverizing chitin-threads, granules or flakes.

**Claims for the following Contracting State : ES**

1. A process for the manufacture of a biological filling agent comprising a composite which is made of chitin or a derivative thereof and a reinforcing material, **characterized by** impregnating therein forcing material with chitin or a derivative thereof.

2. A process for the manufacture of a biological filling agent comprising a composite which is made of chitin or a derivative thereof, an antibiotic and a reinforcing material, **characterized by** impregnating the reinforcing material with chitin or a derivative thereof and an antibiotic.

3. A process for the manufacture of a wound-healing agent comprising a cotton-like chitosan product which is an aggregate of thread-like chitosan **characterized by** spinning chitosan to provide threads which are cut, treated in a mixer and then dried.

4. A process for the manufacture of a wound-healing agent comprising a sponge-like product which is made of chitin, chitosan and a derivative thereof, **characterized by** freeze drying an aqueous dispersion of chitin, chitosan and derivatives thereof, or a mixture of these materials and a water-soluble polymer, or a composite of felt- or sponge-like polyester or polyurethane and said dispersion.

5. A process for the manufacture of a wound-healing agent comprising a cotton-like chitin product which is an aggregate of thread-like chitin, **characterized by** pulverizing chitin-threads, granules or flakes.

FIG 1